# EUROPEAN PATENT SPECIFICATION

(11) **EP 3 097 947 B1**
(45) Date of publication and mention of the grant of the patent: **06.03.2019**
(21) Application number: 14879402.7
(22) Date of filing: 11.12.2014
(51) Int. Cl.: A61N 1/36, A61N 1/04, A61H 3/00, A61B 5/103, A61B 5/00, A61B 5/11

(54) **ELECTROSTIMULATOR**
ELEKTROSTIMULATOR
ÉLECTROSTIMULATEUR

(30) Priority: 24.01.2014 JP 2014011584
(43) Date of publication of application: 30.11.2016
(73) Proprietor: Panasonic Intellectual Property Management Co., Ltd., Osaka-shi, Osaka 540-6207 (JP)
(72) Inventor: MIHARA, Izumi, Osaka 540-6207 (JP); ICHIMURA, Ryo, Osaka 540-6207 (JP); INUI, Keita, Osaka 540-6207 (JP)
(74) Representative: Grünecker Patent- und Rechtsanwälte PartG mbB
(86) International application number: PCT/JP2014/006183
(87) International publication number: WO 2015/111110

(56) References cited:
- WO-A1-97/41748
- GB-A- 2 368 019
- GB-A- 2 495 967
- JP-A- H05 293 188
- JP-A- H05 293 188
- JP-A- 2000 279 536
- JP-A- 2004 313 555
- JP-A- 2004 313 555
- JP-A- 2009 050 533
- JP-A- 2009 050 533
- JP-A- 2012 011 102
- JP-A- 2012 011 102
- JP-A- 2013 094 305
- JP-A- 2013 123 532
- KR-A- 20130 056 026
- US-A1- 2003 093 021
- US-A1- 2007 179 561
- US-A1- 2008 134 541
- US-A1- 2010 324 699
- US-A1- 2011 257 764

## Description

The present invention relates to an electric stimulator.

A conventional electric stimulator includes electrodes that are attached to the thighs and the legs. The electrodes are supplied with current to stimulate the muscles of the thigh and the leg.

Patent Document 1 discloses an example of a conventional electric stimulator that electrically stimulates the thighs and the legs when the user extends his or her knee joints from a bent state while seated in a chair and bends his or her knee joints from an extended state while lying on his or her stomach. Further details concerning electric stimulation during walking and the detection of walking states can be found in Patent Documents 2 to 7.

### PATENT DOCUMENT

Patent Document 1: Japanese Laid-Open Patent Publication No. 2000-279536
Patent Document 2: JP 2004 313555
Patent Document 3: JP 2013 123532
Patent Document 4: JP 2013 094305
Patent Document 5: GB 2 495 967
Patent Document 6: US 2007/179561
Patent Document 7: GB 2 368 019

It is an object of the present invention to provide an electric stimulator that reduces knee pain when walking.

The invention is defined in claim 1. Further aspects and preferred embodiments are defined in the appended claims. Aspects, embodiments and examples of the present disclosure which do not fall under the scope of the appended claims do not form part of the invention and are merely provided for illustrative purposes.

The inventors of the present application have found that when the lower limb of a user is electrically stimulated at a suitable time, knee pain is reduced when the user walks.

One aspect of the present invention provides an electric stimulator according to claim 1.

In the structure of the electric stimulator, the muscle extending across the knee joint is electrically stimulated during the mid-stance phase when knee pain easily occurs. This reduces knee pain when walking. Other aspects and advantages will become apparent from the following description and the accompanying drawings that illustrate the examples of the technical ideas according to the present invention.
Fig. 1 is a block diagram showing an electric stimulator of an embodiment.
Figs. 2A to 2C are schematic views each showing positions of electrodes.
Fig. 3 is a graph showing the relationship of a detection signal and a gait determination in the embodiment.
Fig. 4 is a table showing the relationship of walking modes and electrical stimulation performed by electrodes in the embodiment.
Fig. 5 is a graph showing the relationship of each period of a single walking cycle and the degree of knee pain in the embodiment.
Fig. 6 is a graph showing the relationship of an acceleration area of a thigh and whether or not electrical stimulation is performed in the embodiment.
Fig. 7 is a table showing a list of energizing patterns of another embodiment.

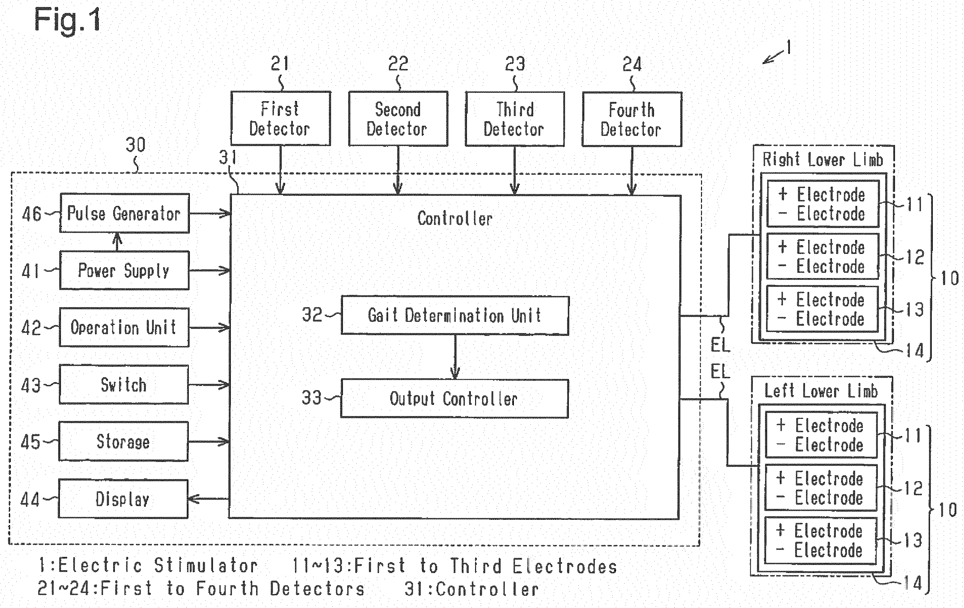

The configuration of an electric stimulator 1 will now be described with reference to Fig. 1.

The electric stimulator 1 includes two stimulators 10, a first detector 21, a second detector 22, a third detector 23, a fourth detector 24, and an operation device 30.

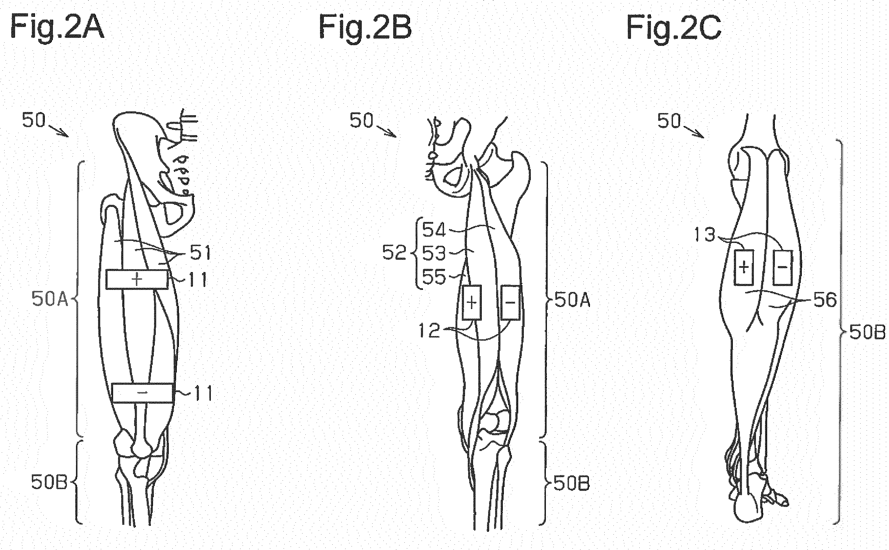

One of the stimulators 10 has a structure corresponding to a right lower limb. The other stimulator 10 has a structure corresponding to a left lower limb. The stimulators 10 electrically stimulate a lower limb 50 (refer to Fig. 2). The stimulators 10 each include a first electrode 11, a second electrode 12, a third electrode 13, and a supporter 14. Each of the electrodes 11 to 13 is attached to a rear surface of the supporter 14 and connected to a controller 31 of the operation device 30 by an electric wire EL. The electrodes 11 to 13 may be referred to as lower limb electrodes. The first electrode 11 corresponds to a "ventral muscle electrode." The second electrode 12 corresponds to a "dorsal muscle electrode." The third electrode 13 corresponds to a "gastrocnemius muscle electrode." The supporter 14 corresponds to a "mounting unit."

The first to fourth detectors 21 to 24 are electrically connected to the controller 31.

One example of the first detector 21 is a pressure sensor. The first detector 21 is attached to the toes of both feet. The first detector 21 outputs, to the controller 31, a detection signal that is in accordance with the pressure generated when the toes contact the ground.

One example of the second detector 22 is a pressure sensor. The second detector 22 is attached to the heels of two feet. The second detector 22 outputs, to the controller 31, a detection signal that is in accordance with the pressure generated when the heels contact the ground.

One example of the third detector 23 is a gyro sensor. The third detector 23 is attached to the side surfaces or front surfaces of both legs above the knees. The third detector 23 outputs, to the controller 31, a detection signal that is in accordance with the angular velocity of the thigh about the hip joint.

One example of the fourth detector 24 is a gyro sensor. The fourth detector 24 is attached to the side surfaces or front surfaces of both legs below the knees. The fourth detector 24 outputs, to the controller 31, a detection signal that is in accordance with the angular velocity of the leg about the knee joint.

The operation device 30 includes the controller 31, a power supply 41, an operation unit 42, a switch 43, a display 44, a memory 45, and a pulse generator 46. The power supply 41, the operation unit 42, the switch 43, the display 44, the memory 45, and the pulse generator 46 are electrically connected to the controller 31.

The controller 31 includes a gait determination unit 32 and an output controller 33.

The gait determination unit 32 determines whether a walking motion of a user correponds to an early stance phase, a mid-stance phase, a late stance phase, an early swing phase, or a late swing phase of a single walking cycle based on the signals of the detectors 21 to 24. The gait determination unit 32 outputs, to the output controller 33, a motion detection signal including information related to a determination result.

The early stance phase refers to a period from when the heel contacts the ground to when the toes contact the ground. The mid-stance phase refers to a period during which the entire sole of the foot is in contact with the ground. The late stance phase refers to a period from when the heel contacting the ground leaves the ground to when the toes contacting the ground leave the ground. The early swing phase refers to a period from when the toes contacting the ground leave the ground to when the raised leg is lowered. The late swing phase refers to a period from when the raised leg is lowered to when the heel separated from the ground contacts the ground.

The output controller 33 is configured by a program or a circuit. The output controller 33 provides the electrodes 11 to 13 with voltage pulses based on motion detection signals of the operation unit 42, the switch 43, and the gait determination unit 32. The output controller 33 changes the widths and intervals of the voltage pulses to control the amount and frequency of the current supplied to each of the electrodes 11 to 13. The output controller 33 supplies current to the electrodes 11 to 13 to output electrical stimulation from the electrodes 11 to 13. The output controller 33 controls the current supplied to the electrodes 11 to 13 in modes including a flat ground walking mode, a stair ascending mode, and a stair descending mode.

The power supply 41 supplies power to the pulse generator 46 and the controller 31. The operation unit 42 includes a switch used to activate and deactivate the controller 31. The operation unit 42 further includes a switch and a dial used to perform a variety of settings. The switch 43 switches the control mode of the output controller 33. The switch 43 includes a plurality of operation pieces corresponding to a plurality of control modes. The display 44 shows the information or the like that indicates the muscle to which electrical stimulation is applied and the intensity of the electrical stimulation. The memory 45 stores the program that controls each of the electrodes 11 to 13 in advance. The pulse generator 46 outputs a signal having a frequency of 20 Hz to 100 Hz, preferably, 40 Hz, to the controller 31.

The muscles of the lower limb 50 will now be described with reference to Fig. 2.

A neutral movement state refers to a state in which the user is extending his or her knee joint. The bending of a knee joint from the neutral movement state and the returning of the bent knee joint to the neutral movement state is referred to as movement of the knee joint.

A neutral rotation state refers to a state in which the knee joint is not rotated about the axis of the lower limb 50.

Rotation of the knee joint refers to rotation of the knee joint about the axis of the lower limb 50 from the neutral rotation state. Rotation of the knee joint from the inner side to the outer side is referred to as "outer rotation," and rotation of the knee joint from the outer side to the inner side is referred to as "inner rotation."

The lower limb 50 is divided into the thigh 50A and the leg 50B. The muscles that form the thigh 50A include the quadriceps femoris muscle 51, which serves as a lower limb ventral muscle group, and the hamstring 52, which serves as a lower limb dorsal muscle group. The hamstring 52 includes the semitendinosus muscle 53, the biceps femoris muscle 54, and the semimembranosus muscle 55. The muscles that form the leg 50B include a gastrocnemius muscle 56.

The quadriceps femoris muscle 51, the hamstring 52, and the gastrocnemius muscle 56 extend across the knee joint. When the quadriceps femoris muscle 51 and the hamstring 52 contract, the knee joint is pulled toward the upper side so that force acts on the knee joint to return the knee joint to the neutral movement state. When the gastrocnemius muscle 56 contracts, the knee joint is pulled toward the lower side so that force acts on the knee joint to return the knee joint to the neutral movement state. Thus, when at least one of the quadriceps femoris muscle 51, the hamstring 52, and the gastrocnemius muscle 56 contracts, force act on the knee joint in a direction that reduces the pivoting of the knee joint.

The quadriceps femoris muscle 51 and the hamstring 52 contribute to rotation of the knee joint. When the quadriceps femoris muscle 51 and the hamstring 52 contract, force acts on the knee joint to return the state in which the knee joint is rotated outwardly or inwardly to the neutral rotation state. Thus, when at least one of the quadriceps femoris muscle 51 and the hamstring 52 contracts, force acts on the knee joint in a direction that reduces rotation of the knee joint.

The positional relationships of the muscles of the lower limb 50 and the electrodes 11 to 13 will now be described. Fig. 2 shows the positional relationships of the muscles of the right lower limb 50 and the electrodes 11 to 13. The positional relationships of the muscles of the left lower limb and the electrodes 11 to 13 are the same as the positional relationship of the muscles of the right lower limb 50 and the electrodes 11 to 13.

The first electrodes 11 are attached to portions of the supporter 14 (refer to Fig. 1) corresponding to the quadriceps femoris muscle 51. When the supporter 14 is attached to the right lower limb, a positive pole and a negative pole of the first electrode 11 are spaced apart from each other in the vertical direction on the quadriceps femoris muscle 51. The upper electrode may be arranged to include a motor point (hereinafter referred to as MT position) of the rectus femoris muscle, and the lower electrode may be arranged to include an MT position of the vastus medialis muscle and an MT position of the vastus lateralis muscle. The second electrodes 12 are attached to portions of the supporter 14 corresponding to the hamstring 52. When the supporter 14 is attached to the right lower limb, a positive pole and a negative pole of the second electrode 12 are spaced apart from each other in the sideward direction at the vertically middle part of the hamstring 52. One of the two electrodes may be arranged to include an MT of the long head of the biceps femoris muscle and an MT of the semitendinosus muscle. The third electrodes 13 are attached to portions of the supporter 14 corresponding to the gastrocnemius muscle 56. When the supporter 14 is attached to the right lower limb, a positive pole and a negative pole of the third electrode 13 are spaced apart from each other in the sideward direction of the gastrocnemius muscle 56. One of the two electrodes may be arranged to include an MT of the medial head of the gastrocnemius muscle and an MT of the lateral head of the gastrocnemius muscle.

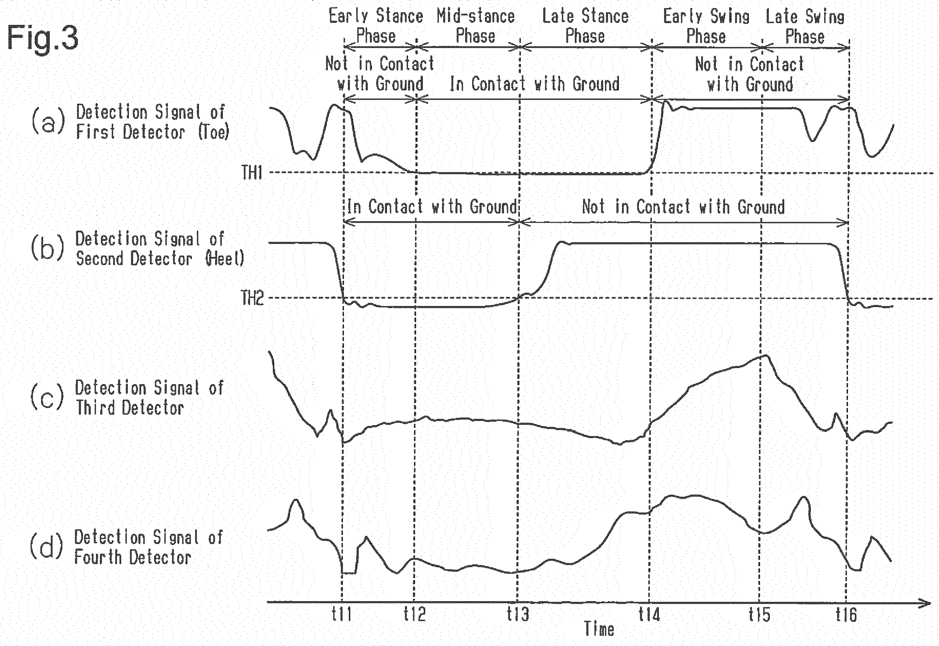

Gait determination will now be described with reference to Fig. 3.

When the walking phase is the early stance phase, the toes are not in contact with the ground, and the heel is in contact with the ground. Thus, as shown in the period from time t11 to time t12, the detection signal of the first detector 21 indicates a value that is greater than or equal to a first threshold value TH1, and the detection signal of the second detector 22 indicates a value that is less than a second threshold value TH2. Thus, the gait determination unit 32 determines at, for example, time t11 that the walking phase is the early stance phase because the detection signal of the first detector 21 indicates a value that is greater than or equal to the first threshold value TH1 and the detection signal of the second detector 22 indicates a value that is less than the second threshold value TH2. When determining that the walking phase is the early stance phase, the gait determination unit 32 outputs an early stance phase detection signal to the output controller 33 as a motion detection signal.

When the walking phase is the mid-stance phase, the toes and the heel are in contact with the ground. Thus, as shown in the period from time t12 to time t13, the detection signal of the first detector 21 indicates a value that is less than the first threshold value TH1, and the detection signal of the second detector 22 indicates a value that is less than the second threshold value TH2. Thus, the gait determination unit 32 determines at, for example, time t12 that the walking phase is the mid-stance phase because the detection signal of the first detector 21 indicates a value that is less than the first threshold value TH1 and the detection signal of the second detector 22 indicates a value that is less than the second threshold value TH2. When determining that the walking phase is the mid-stance phase, the gait determination unit 32 outputs a mid-stance phase detection signal to the output controller 33 as a motion detection signal.

When the walking phase is the late stance phase, the toes are in contact with the ground, and the heel is not in contact with the ground. Thus, as shown in the period from time t13 to time t14, the detection signal of the first detector 21 indicates a value that is less than the first threshold value TH1, and the detection signal of the second detector 22 indicates a value that is greater than or equal to the second threshold value TH2. Thus, the gait determination unit 32 determines at, for example, time t13 that the walking phase is the late stance phase because the detection signal of the first detector 21 indicates a value that is less than the first threshold value TH1 and the detection signal of the second detector 22 indicates a value that is greater than or equal to the second threshold value TH2. When determining that the walking phase is the late stance phase, the gait determination unit 32 outputs a late stance phase detection signal to the output controller 33 as a motion detection signal.

When the walking phase is the swing phase, the toes and the heel are not in contact with the ground. Thus, as shown in the period from time t14 to time t16, the detection signal of the first detector 21 indicates a value that is greater than or equal to the first threshold value TH1, and the detection signal of the second detector 22 indicates a value that is greater than or equal to the second threshold value TH2. Further, when the walking phase is the early swing phase, the angular speed change of the thigh 50A is larger than the angular speed change of the leg 50B. Thus, as shown in the period from time t14 to time t15, the value of a detection signal of the third detector 23 increases, and the value of a detection signal of the fourth detector 24 decreases. Accordingly, the gait determination unit 32 determines at, for example, time t14 that the walking phase is the early swing phase because the detection signal of the first detector 21 indicates a value that is less than the first threshold value TH1 and the detection signal of the second detector 22 indicates a value that is less than the second threshold value TH2. Further, the gait determination unit 32 determines that the walking phase is the swing phase based on the facts that the value of the detection signal of the third detector 23 increases and that the value of the detection signal of the fourth detector 24 decreases. When determining that the walking phase is the early swing phase, the gait determination unit 32 outputs an early swing phase detection signal to the output controller 33 as a motion detection signal.

When the walking phase is the late swing phase, the angular speed change of the leg 50B is larger than the angular speed change of the thigh 50A. Thus, as shown in time t15 to a predetermined period, the value of the detection signal of the third detector 23 decreases, and the value of the detection signal of the fourth detector 24 increases. Thus, the gait determination unit 32 determines at, for example, time t15 that the walking phase is the late swing phase because the value of the detection signal of the third detector 23 decreases and that the value of the detection signal of the fourth detector 24 increases. When determining that the walking phase is the late swing phase, the gait determination unit 32 outputs a late swing phase detection signal to the output controller 33 as a motion detection signal.

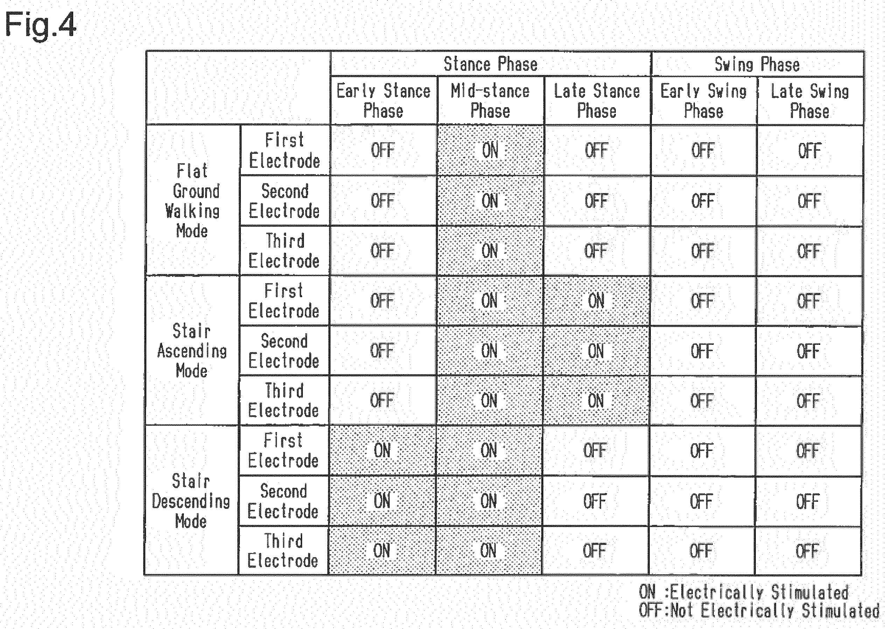

The output modes of electrical stimulation of the electrodes 11 to 13 (refer to Fig. 1) in the single walking cycle will now be described with reference to Fig. 4. In the following description with reference to Fig. 4, the components of the electric stimulator 1 to which reference numbers are added indicate the components shown in Fig. 1.

The output controller 33 performs the following energizing patterns on the electrodes 11 to 13 in the flat ground walking mode, the stair ascending mode, and the stair descending mode.

When the flat ground walking mode is selected with the switch 43, the output controller 33 has each of the electrodes 11 to 13 output electrical stimulation during the mid-stance phase, and the output controller 33 does not have each of the electrodes 11 to 13 output electrical stimulation during the early stance phase, the late stance phase, the early swing phase, and the late swing phase.

When the stair ascending mode is selected with the switch 43, the output controller 33 has each of the electrodes 11 to 13 output electrical stimulation during the mid-stance phase and the late stance phase, and the output controller 33 does not have each of the electrodes 11 to 13 output electrical stimulation during the early stance phase, the early swing phase, and the late swing phase.

When the stair descending mode is selected with the switch 43, the output controller 33 has each of the electrodes 11 to 13 output electrical stimulation during the early stance phase and the mid-stance phase, and the output controller 33 does not have each of the electrodes 11 to 13 output electrical stimulation during the late stance phase, the early swing phase, and the late swing phase.

The inventors of the present application measured the degree of knee pain and the variation amount of rotation of the knee joint based on whether or not the electric stimulator 1 performs electrical stimulation. The inventors of the present application measured the degree of knee pain of the user when walking, with the first electrode 11 and the second electrode 12 attached to the user, and the third electrode 13 not attached to the user. The inventors of the present application measured the pivoting amount of the knee joint in the same manner.

The measurement conditions and the measurement results of the degree of knee pain will now be described.

The inventors of the present application conducted interviewed a plurality of subjects about the degree of knee pain in the early stance phase, the mid-stance phase, the late stance phase, and the swing phase. The pain was compared with the motion in which the knee pain was most painful in daily life motions.

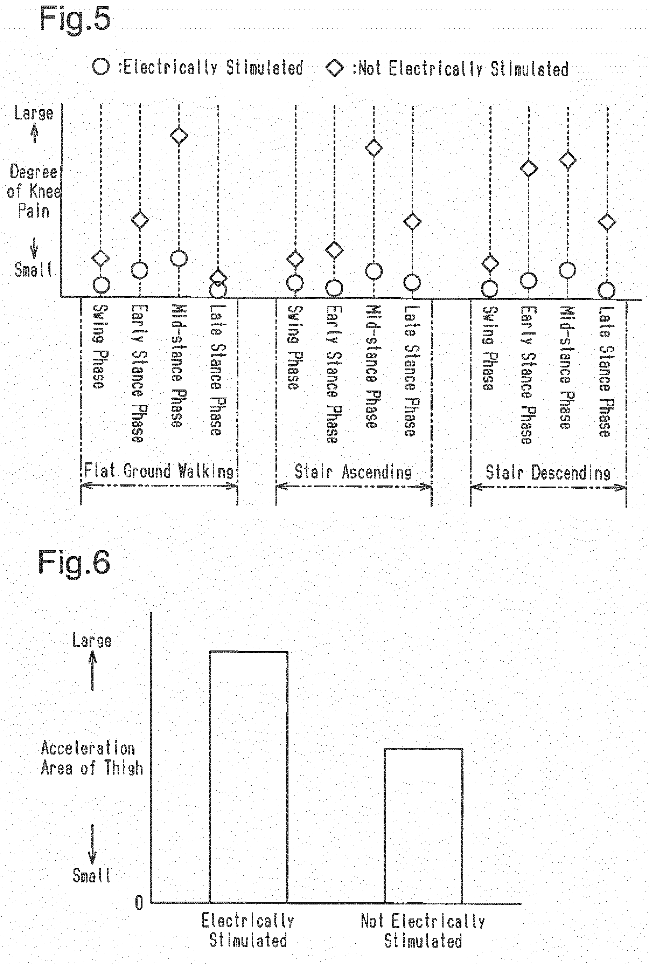

The inventors of the present application obtained the following measurement results as shown in Fig. 5.

When the subject walks on flat ground, as the walking motion, without receiving electrical stimulation, the degree of knee pain is larger in the order of the mid-stance phase, the early stance phase, the swing phase, and the late stance phase. When the subject ascends as the walking motion without receiving electrical stimulation, the degree of knee pain is larger in the order of the mid-stance phase, the late stance phase, the early stance phase, and the swing phase. When the subject descends as the walking motion without receiving electrical stimulation, the degree of knee pain is larger in the order of the mid-stance phase, the early stance phase, the late stance phase, and the swing phase.

The inventors of the present application have found through the measurement results that the degree of knee pain when electrical stimulation is applied during the stance phase and the swing phase is smaller than the degree of knee pain when electrical stimulation is not applied during the stance phase and the swing phase. Further, the inventors of the present application have found that the degree of knee pain when electrical stimulation is applied during the mid-stance phase is much smaller than the degree of knee pain when electrical stimulation is not applied during the mid-stance phase.

The measurement conditions and the measurement results of the degree of rotation of the knee joint will now be described.

The inventors of the present application measured an acceleration area of the thigh 50A as the pivoting amount of the knee joint under the measurement condition shown in Fig. 6.

A triaxial accelerometer (not shown) was attached to the left and right thighs 50A of the subject. The triaxial accelorometer outputs, to an analysis computer (not shown), a signal that is in accordance with the accleeration of the thigh 50A. The analysis computer calculated an acceleration area, which is the area of an acceleration waveform of a sideward component during the stance phase.

The meausrement results show that the acceleration area of the thigh 50A when the user walks while being electrically stimulated is smaller than the acceleration area of the thigh 50A when the user walks without being electrically stimulated. It is understood that this is because the electric stimulator 1 electrically stimulates and contracts the quadriceps femoris muscle 51 and the hamstring 52 to limit the pivoting amount of the knee joint.

The operation of the electric stimulator 1 of the present embodiment will now be described.

The electric stimulator 1 electrically stimulates the quadriceps femoris muscle 51, the hamstring 52, and the gastrocnemius muscle 56. This contracts the quadriceps femoris muscle 51 and the hamstring 52 and thus limits rotation of the knee joint during the mid-stance phase. Further, this contracts the gastrocnemius muscle 56 and limits movement of the knee joint.

The electric stimulator 1 of the present embodiment has the advantages described below.
(1) The electric stimulator 1 electrically stimulates the muscles extending across the knee joint during the mid-stance phase. Thus, the knee pain that occurs when walking is reduced. Further, the electric stimulator 1 does not electrically stimulate the muscles extending across the knee joint during the swing phase, in which the load to the knee decreases when the user walks. Thus, increases in the knee pain and the muscle fatigue caused by electrical stimulation are reduced. Accordingly, the electric stimulator 1 reduces occurrence of knee pain and increase in muscle fatigue when walking.
(2) In the stair ascending mode, the electric stimulator 1 has each of the electrodes 11 to 13 output electrical stimulation during the late stance phase. This reduces knee pain.
(3) In the stair descending mode, the electric stimulator 1 has each of the electrodes 11 to 13 output electrical stimulation during the early stance phase. This reduces knee pain.
(4) The electric stimulator 1 electrically stimulates the quadriceps femoris muscle 51, the hamstring 52, and the gastrocnemius muscle 56 during the mid-stance phase. This further reduces knee pain.
(5) The electric stimulator 1 does not electrically stimulate the quadriceps femoris muscle 51, the hamstring 52, and the gastrocnemius muscle 56 during the swing phase. This limits increases in muscle fatigue caused by electrical stimulation.

The present electric stimulator may be modified as described below.

The first electrodes 11 attached to the supporter 14 may be spaced apart from each other in the sideward direction within the portion of the supporter 14 corresponding to the quadriceps femoris muscle 51.

The positions of the second electrodes 12 attached to the supporter 14 may be changed to positions other than the vertically middle part of the hamstring 52 within the portion of the supporter 14 corresponding to the hamstring 52.

The attached third electrodes 13 may be spaced apart from each other in the vertical direction within the portion of the supporter 14 corresponding to the gastrocnemius muscle 56.

In addition to the electodes 11 to 13, electrodes may be attached to portions of the supporter 14 corresponding to the following portions of the lower limb 50.
(A1) The electrodes are attached to a portion of the supporter 14 corresponding to the outer side of the leg 50B. This allows the electrodes to electrically stimulate the dorsal muscles of the foot.
(A2) The attached electrodes are spaced apart from each other in the vertical direction within the portion of the supporter 14 corresponding to the gluteus medius muscle.
(A3) The attached electrodes are spaced apart from each other in the vertical direction within the portion of the supporter 14 corresponding to the gluteus maximus muscle.

In the strctures of (A2) and (A3), when the gluteus medius muscle and the gluteus maximus muscle are electrically stimulated and contracted, the hamstring 52 contracts in cooperation. This limits rotation of the knee joint.

The electric stimulator 1 may include at least one of the electrode of (A2) or the electrode of (A3) instead of the second electrode 12.

One or two of the electrodes 11 to 13 may be omitted.

In the flat ground walking mode, the stair ascending mode, and the stair descending mode, each of the electrodes 11 to 13 may electrically stimulate the muscles extending across the knee joint during a certain period of the mid-stance phase.

In the stair ascending mode, each of the electrodes 11 to 13 may electrically stimulate the muscles extending across the knee joint during a certain period of the late stance phase.

In the stair descending mode, each of the electrodes 11 to 13 may electrically stimulate the muscles extending acorss the knee joint during a certain period of the early stance phase.

The controller 31 may output electrical stimulation to each of the electrodes 11 to 13 during a certain period of the early swing phase and a certain period of the late swing phase.

In the early swing phase, the controller 31 may output weaker electrical stimulation to each of the electrodes 11 to 13 than the electrical stimulation output to each of the electrodes 11 to 13 during the mid-stance phase. The methods for generating the weak electrical stimulation include, for example, (B1) to (B3) described below. The following (B1) to (B3) may be combined with one another.
(B1) Electrical stimulation is output to one or two of the electrodes 11 to 13.
(B2) The period in which electrical stimulation is output during the early swing phase is shorter than the period in which electrical stimulation is output during the mid-stance phase.
(B3) Small current is supplied to the one of the electrodes 11 to 13 that outputs electrical stimulation.

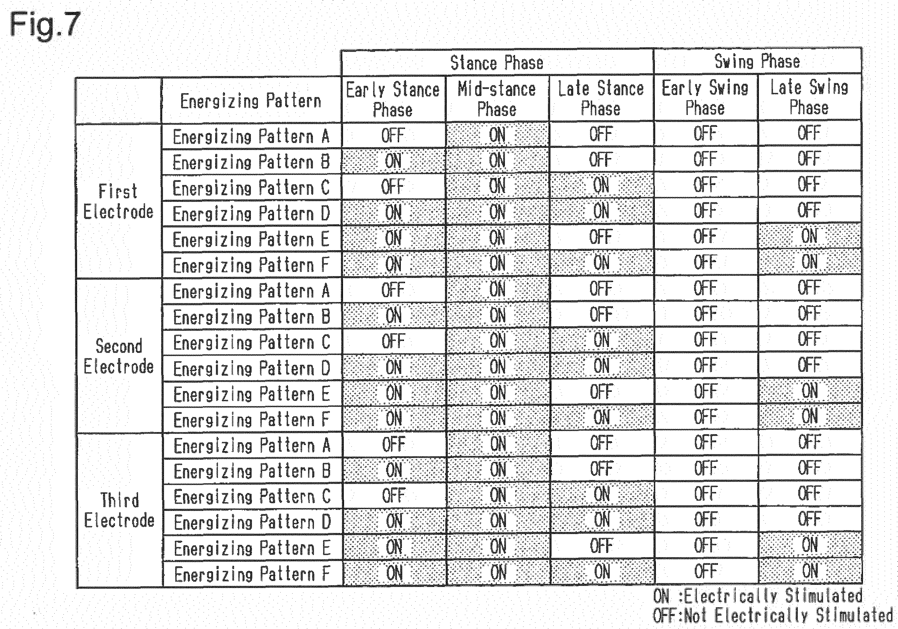

The controller 31 may include energizing patterns shown in Fig. 7.
(a1) Energizing pattern A has the electrodes output electrical stimulation during the mid-stance phase and does not have the electrodes output electrical stimulation during the early stance phase, the late stance phase, and the swing phase.
(a2) Energizing pattern B has the electrodes output electrical stimulation during the early stance phase and the mid-stance phase and does not have the electrodes output electrical stimulation during the late stance phase and the swing phase.
(a3) Energizing pattern C has the electrodes output electrical stimulation during the mid-stance phase and the late stance phase and does not have the electrodes output electrical stimulation during the early stance phase and the swing phase.
(a4) Energizing pattern D has the electrodes output electrical stimulation during the stance phase and does not have the electrodes output electrical stimulation during the swing phase.
(a5) Energizing pattern E has the electrodes output electrical stimulation during the early stance phase, the mid-stance phase, and the late swing phase and does not have the electrodes output electrical stimulation during the late stance phase and the early swing phase.
(a6) Energizing pattern F has the electrodes output electrical stimulation during the stance phase and the late swing phase and does not have the electrodes output electrical stimulation during the early stance phase.

In addition to advantages (1) to (5) of the above embodiment, the energizing patterns obtain advantages (6) and (7).
(6) Energizing pattern D lengthens the period that limits rotation of the knee joint when the sole of the foot contacts the ground. This reduces the occurrence of knee pain during the stance phase. In addition, a combination of energizing patterns D further reduces the occurrence of knee pain and easily strengthens the muscles that extend across the knee joint. It is thus expected that the user becomes physically strong.
(7) In energizing pattern E or F, the muscles extending across the knee joint are electrically stimulated before the heel contacts the ground. Thus, when the heel starts to contact the ground, the state in which electrical stimulation has been already performed is easily formed. This reduces the occurrence of knee pain when the heel contacts the ground.

The controller 31 may control at least one of the electrodes 11 to 13 based on energizing patterns A to F. In this case, the controller 31 may have at least one of the configurations of (C1) and (C2) described below.
(C1) The controller 31 does not have one or two of the electrodes 11 to 13 output electrical stimulation during the mid-stance phase.
(C2) The controller 31 has one or two of the electrodes 11 to 13 output electrical stimulation during the early swing phase.

The gait determination unit 32 may determine the early stance phase, the mid-stance phase, the late stance phase, the early swing phase, and the late swing phase with only the third detector 23.

When the walking phase is the early swing phase, the value of the detection signal of the third detector 23 gradually increases. Thus, the gait determination unit 32 determines the early stance phase when the change speed of the detection signal of the third detector 23 is a positive value that indicates increase, when the absolute value of the speed change is less than or equal to a predetermined speed threshold, and when the variation amount of the detecion signal of the third detector 23 is larger than a change threshold. The change speed of the detection signal of the third detector 23 is calculated by differentiating the obtained detection signal. The variation amount of the detecion signal of the third detector 23 is calculated from, for example, the differecne of a detection signal that is sampled this time and a detection singal that was sampled a plurality of times before. The change threshold is set in advance through tests or the like.

When the walking phase is the mid-stance phase, the detection signal of the third detector 23 does not change substantially. Thus, the gait determination unit 32 determines the mid-stance phase when the absolute value of the speed change of the detection signal of the third detector 23 is less than or equal to the speed threshold and when the variation amount of the detecion signal of the third detector 23 is less than or equal to the change threshold.

When the walking phase is the late stance phase, the detection signal of the third detector 23 gradually decreases. Thus, the gait determination unit 32 determines the late stance phase when the change speed of the detection signal of the third detector 23 is a negative value that indicates decrease, when the absolute value of the speed change is less than or equal to the speed threshold, and when the variation amount of the detecion signal of the third detector 23 is larger than the change threshold.

When the walking phase is the early swing phase, the detection signal of the third detector 23 increases sharply. Thus, the gait determination unit 32 determines the early swing phase when the change speed of the detection signal of the third detector 23 is a positive value and when the absolute value of the speed change is larger than the speed threshold.

When the walking phase is the late swing phase, the detection signal of the third detector 23 decreases sharply. Thus, the gait determination unit 32 determines the late swing phase when the change speed of the detection signal of the third detector 23 is a negative value and when the absolute value of the speed change is larger than the speed threshold.

The gait determination unit 32 may determine the early stance phase, the mid-stance phase, the late stance phase, the early swing phase, and the late swing phase with only the fourth detector 24.

When the walking phase is the early swing phase, the value of the detection signal of the fourth detector 24 gradually decreases. Thus, the gait determination unit 32 determines the early stance phase when the change speed of the detection signal of the fourth detector 24 is a negative value that indicates decrease, when the absolute value of the speed change is less than or equal to a predetermined speed threshold, and when the variation amount of the detecion signal of the fourth detector 24 is larger than a change threshold. The change speed and the variation amount of the detecion signal of the fourth detector 24 are calculated in the same manner as the change speed and the variation amount of the detection signal of the third detector 23.

When the walking phase is the mid-stance phase, the detection signal of the fourth detector 24 does not change substantially. Thus, the gait determination unit 32 determines the mid-stance phase when the absolute value of the speed change of the detection signal of the fourth detector 24 is less than or equal to the speed threshold and when the variation amount of the detecion signal of the fourth detector 24 is less than or equal to the change threshold.

When the walking phase is the late stance phase, the detection signal of the fourth detector 24 increases sharply. Thus, the gait determination unit 32 determines the late stance phase when the change speed of the detection signal of the fourth detector 24 is a positive value that indicates increase and when the absolute value of the speed change is larger than the speed threshold.

When the walking phase is the early swing phase, the detection signal of the fourth detector 24 gradually increases. Thus, the gait determination unit 32 determines the early swing phase when the change speed of the detection signal of the third detector 23 is a positive value, when the absolute value of the speed change is larger than the speed threshold, and the variation amount of the detection signal of the fourth detector 24 is larger than the change threshold.

When the walking phase is the late swing phase, the detection signal of the fourth detector 24 decreases sharply. Thus, the gait determination unit 32 determines the late swing phase when the change speed of the detection signal of the third detector 23 is a negative value and when the absolute value of the speed change is less than or equal to the speed threshold.

The gait determination unit 32 may use a means other than the detection signals of the first detector 21 to the fourth detector 24 to determine the early stance phase, the mid-stance phase, the late stance phase, the early swing phase, and the late swing phase of the single walking cycle. The gyro sensor of the thigh may be located on the back surface of the thigh, and the gyro sensor of the lower limb may be located on the back surface of the lower limb. However, it is more difficult for the back surface of the thigh and the back surface of the lower limb to be vertical to the ground than the ventral side and the side surface, and the angles relative to the ground differ between individuals. Thus, in the embodiment, the gyro sensors of the thigh and the leg are located on the ventral side and side surface.

Further, a foot pressure sensor is capable of sensing when the gyro sensor is attached to the top or the bottom surface (arch) of the foot. In the early swing phase, the angular velocity detected by the gyro sensor increases due to a sudden swing of the foot to the forward direction during walking. In the late swing phase, the angular velocity decreases. In the early stance phase, the detection signal receives vibration when coming into contact with the ground at the angular speed of 0, and the detected angular velocity increases in the opposite direction of that of the swing phase when the entire surface of the foot contacts the ground. In the late stance phase, the angular velocity further increases. When the gyro sensor of the foot detects this state and performs threshold value determination, the gyro sensor serves as an alternative of the foot pressure sensor. For example, the gait determination unit 32 determines the early stance phase, the mid-stance phase, the late stance phase, the early swing phase, and the late swing phase of the single walking cycle from an arm swing or trunk tilt of the user. In this case, the gyro sensor is attached to the arm or trunk of the user.

The pulse generator 46 may output a signal having a high frequency of 3 kHZ or greater to the controller 31. The controller 31 sets a period in which electrical stimulation of 3 kHz or greater is output to each of the electrodes 11 to 13 during the mid-stance phase and a period in which electrical stimulation is not output to each of the electrodes 11 to 13 during the mid-stance phase. The period in which electrical stimulation is output to each of the electrodes 11 to 13 is shorter than the period in which electrical stimulation is not output to each of the electrodes 11 to 13. When electrical stimulation having such a high frequency of 3 kHz or greater is given to the muscles extending the knee joint, the sensory nerve does not respond. Thus, the pain is reduced. This increases the effect of reducing knee pain when walking. The early stance phase of the stair descending mode and the late stance phase of the stair ascending mode may be set in the same manner. Further, the early stance phase, the late stance phase, and the late swing phase of the controller 31 of the modified examples of Fig. 7 may be set in the same manner.

The memory 45 may be a computer-readable recording medium such as ROM or EEPROM. The gait determination unit 32 and the output controller 33 may be dedicated hardware. Instead, the functions of the gait determination unit 32 and the output controller 33 may be achieved by the processor of the controller 31 executing a computer-readable instruction stored in the memory 45. The memory 45 may be part of the controller 31.

The present invention includes the following examples.
(Clause 1) The electric stimulator according to claim 1, wherein in the swing phase, the controller determines an early swing phase, during which a leg is raised, and a late swing phase, during which the leg is lowered, and has the electrode output electric stimulation during a period from the late swing phase to the mid-stance phase.
(Clause 2) The electric stimulator according to claim 1, wherein in the single walking period, the controller determines an early stance phase, during which a heel is in contact with ground and a foot toe is not in contact with the ground, a late stance phase, during which the foot toe is in contact with the ground and the heel is not in contact with the ground, an early swing phase, during which a leg is raised, and a late swing phase, during which the leg is lowered, and has the electrode output electric stimulation during a period from the late swing phase to the late stance phase.

The foregoing description is to be considered as illustrative and not restrictive. For example, the above embodiments or one or more modifications may be used in combination with each other. The subject matter of the present invention may be included in fewer features than all of the disclosed features of the specific embodiments. The scope of the present invention and equivalence of the present invention are to be understood with reference to the appended claims.

## Claims

1. An electric stimulator (1) comprising:
a controller (31) that at least determines, in a single walking cycle, one of a mid-stance phase (t12-t13), during which an entire foot sole is in contact with ground, and a swing phase (t14-t16), during which the entire foot sole is not in contact with the ground; and
an electrode (10) that outputs electrical stimulation to a muscle extending across a knee joint,
**characterized in that**
the controller (31) includes a period during the mid-stance phase (t12-t13) in which the controller (31) has the electrode (10) output electrical stimulation and a period during the swing phase (t14-t16) in which the controller (31) has the electrode (10) output weaker electrical stimulation than the electrical stimulation output during the mid-stance phase (t12-t13), or
the controller (31) includes a period during the mid-stance phase (t12-t13) in which the controller (31) has the electrode (10) output electrical stimulation and a period during the swing phase (t14-t16) in which the controller (31) does not have the electrode (10) output electrical stimulation.

2. The electric stimulator (1) according to claim 1, wherein
in a single walking cycle, the controller (31) determines an early stance phase (t11-t12), during which a heel is in contact with the ground and a foot toe is not in contact with the ground, and
the controller (31) has the electrode (10) output electrical stimulation during the early stance phase (t11-t12).

3. The electric stimulator (1) according to claim 1 or 2, wherein
in a single walking cycle, the controller (31) determines a late stance phase (t13-t14), during which the foot toe is in contact with the ground and the heel is not in contact with the ground, and
the controller (31) has the electrode (10) output electrical stimulation during the late stance phase (t13-t14).

4. The electric stimulator (1) according to any one of claims 1 to 3, wherein
in the swing phase (t14-t16), the controller (31) determines an early swing phase (t14-t15), during which a leg is raised, and a late swing phase (t15-t16), during which the leg is lowered, and
the controller (31) includes a period in which the controller (31) has the electrode (10) output electrical stimulation during the late swing phase (t15-t16) and a period in which the controller (31) has the electrode (10) output weaker electrical stimulation than the electrical stimulation output during the mid-stance phase (t12-t13), or
the controller (31) includes a period in which the controller (31) has the electrode (10) output electrical stimulation during the late swing phase (t15-t16) and a period in which the controller (31) does not have the electrode (10) output electrical stimulation during the early swing phase (t14-t15).

5. The electric stimulator (1) according to any one of claims 1 to 4, comprising a plurality of the electrodes (11, 12, 13) and a mounting unit (14), wherein:
the mounting unit (14) is an object mounted on a lower limb;
one of the electrodes (11, 12, 13) is a ventral muscle electrode (11) attached to a portion of the mounting unit (14) corresponding to a lower limb ventral muscle group, which is a muscle extending across the knee joint;
one of the electrodes (11, 12, 13) is a dorsal muscle electrode (12) attached to a portion of the mounting unit (14) corresponding to a lower limb dorsal muscle group, which is a muscle extending across the knee joint;
one of the electrode (13)s is a gastrocnemius muscle electrode (13) attached to a portion of the mounting unit (14) corresponding to a lower limb gastrocnemius muscle group, which is a muscle extending across the knee joint;
in the single walking cycle, the controller (31) determines an early stance phase (t11-t12), during which a heel is in contact with the ground and a foot toe is not in contact with the ground, a late stance phase (t13-t14), during which the foot toe is in contact with the ground and the heel is not in contact with the ground, an early swing phase (t14-t15), during which a leg is raised, and a late swing phase (t15-t16), during which the leg is lowered;
the controller (31) has at least one of the electrodes (11, 12, 13) output electrical stimulation during the early stance phase (t11-t12) or does not have at least one of the electrodes (11, 12, 13) output electrical stimulation during the early stance phase (t11-t12);
the controller (31) has at least one of the electrodes (11, 12, 13) output electrical stimulation during the late stance phase (t13-t14) or does not have at least one of the electrodes (11, 12, 13) output electrical stimulation during the late stance phase (t13-t14); and
the controller (31) has at least one of the electrodes (11, 12, 13) output electrical stimulation during the late swing phase (t15-t16) or does not have at least one of the electrodes (11, 12, 13) output electrical stimulation during the late swing phase (t15-t16).

6. An electric stimulator (1) according to any of claims 1 to 4, wherein
the controller (31) is configured to receive, from a detector, a detection signal that cyclically changes in accordance with a walking cycle;
the electrode is a lower limb electrode (11-13) connected to the controller (31) and configured to output knee joint stimulation under control of the controller (31); and
the electric stimulator (1) further comprises
a computer-readable recording medium connected to or incorporated in the controller (31), wherein the computer-readable recording medium holds a computer-readable instruction configured so that the controller (31):
has the lower limb electrode (11-13) output a first knee joint stimulation having a first intensity whenever the detection signal indicates a mid-stance phase (t12-t13); and
has the lower limb electrode (11-13) output a second knee joint stimulation having a second intensity, which is lower than the first intensity, or stop outputting a knee joint stimulation from the lower limb electrode (11-13) whenever the detection signal indicates a swing phase (t14-t16).

## Patentansprüche

1. Elektrostimulator (1), mit:
einer Steuerung (31), die in einem einzigen Schrittzyklus zumindest eine Mittelstellungsphase (t12-t13), während welcher eine gesamte Fußsohle mit dem Boden in Kontakt ist, oder eine Schwungphase (t14-t16), während welcher die gesamte Fußsohle nicht mit dem Boden in Kontakt ist, ermittelt; und
einer Elektrode (10), die eine elektrische Stimulation an einen Muskel ausgibt, der sich über ein Kniegelenk erstreckt,
**dadurch gekennzeichnet, dass**
die Steuerung (31) eine Zeitdauer während der Mittelstellungsphase (t12-t13) enthält, in der die Steuerung (31) die Elektrode (10) veranlasst, eine elektrische Stimulation auszugeben, und eine Zeitdauer während der Schwungphase (t14-t16) enthält, in der die Steuerung (31) die Elektrode (10) veranlasst, eine schwächere elektrische Stimulation als die elektrische Stimulation auszugeben, die während der Mittelstellungsphase (t12-t13) ausgegeben wird, oder
die Steuerung (31) eine Zeitdauer während der Mittelstellungsphase (t12-t13) enthält, in der die Steuerung (31) die Elektrode (10) veranlasst, eine elektrische Stimulation auszugeben, und eine Zeitdauer während der Schwungphase (t14-t16) enthält, in der die Steuerung (31) die Elektrode (10) veranlasst, eine elektrische Stimulation nicht auszugeben.

2. Elektrostimulator (1) nach Anspruch 1, wobei
in einem einzigen Schrittzyklus die Steuerung (31) eine Frühstellungsphase (t11-t12) ermittelt, während welcher eine Ferse mit dem Boden in Kontakt ist und eine Fußzehe mit dem Boden nicht in Kontakt ist, und
die Steuerung (31) die Elektrode (10) veranlasst, eine elektrische Stimulation während der Frühstellungsphase (t11-t12) auszugeben.

3. Elektrostimulator (1) nach Anspruch 1 oder 2, wobei
in einem einzigen Schrittzyklus die Steuerung (31) eine Spätstellungsphase (t13-t14) ermittelt, während welcher die Fußzehe mit dem Boden in Kontakt ist und die Ferse mit dem Boden nicht in Kontakt ist, und
die Steuerung (31) die Elektrode (10) veranlasst, eine elektrische Stimulation während der Spätstellungsphase (t13-t14) auszugeben.

4. Elektrostimulator (1) nach einem der Ansprüche 1 bis 3, wobei
in der Schwungphase (t14-t16) die Steuerung (31) eine Früh-Schwungphase (t14-t15) ermittelt, während welcher ein Bein angehoben ist, und eine Spät-Schwungphase (t15-t16) ermittelt, während welcher das Bein abgesenkt ist, und
die Steuerung (31) eine Zeitdauer enthält, in der die Steuerung (31) die Elektrode (10) veranlasst, eine elektrische Stimulation während der Spät-Schwungphase (t15-t16) auszugeben, und eine Zeitdauer enthält, in welcher die Steuerung (31) die Elektrode (10) veranlasst, eine schwächere elektrische Stimulation als die elektrische Stimulation auszugeben, die während der Mittelstellungsphase (t12-t13) ausgegeben wird, oder
die Steuerung (31) eine Zeitdauer enthält, in der die Steuerung (31) die Elektrode (10) veranlasst, eine elektrische Stimulation während der Spät-Schwungphase (t15-t16) auszugeben, und eine Zeitdauer enthält, in der die Steuerung (31) die Elektrode (10) veranlasst, eine elektrische Stimulation während der Früh-Schwungphase (t14-t15) nicht auszugeben.

5. Elektrostimulator (1) nach einem der Ansprüche 1 bis 4, mit mehreren der Elektroden (11, 12, 13) und einer Befestigungseinheit (14), wobei:
die Befestigungseinheit (14) ein Objekt ist, das einer unteren Gliedmaße zu befestigen ist;
eine der Elektroden (11, 12, 13) eine Ventralmuskelelektrode (11) ist, die an einem Bereich der Befestigungseinheit (14) angebracht ist, der einer Ventralmuskelgruppe einer unteren Gliedmaße entspricht, die ein Muskel ist, der sich über das Kniegelenk erstreckt;
eine der Elektroden (11, 12, 13) eine dorsale Muskelelektrode (12) ist, die an einem Bereich der Befestigungseinheit (14) angebracht ist, der einer dorsalen Muskelgruppe einer unteren Gliedmaße entspricht, die ein Muskel ist, der sich über das Kniegelenk erstreckt;
eine der Elektroden (13) eine Gastrocnemius-Muskelelektrode (13) ist, die an einem Bereich der Befestigungseinheit (14) angebracht ist, der einer Gastrocnemius-Muskelgruppe einer unteren Gliedmaße entspricht, die ein Muskel ist, der sich über das Kniegelenk erstreckt;
in dem einzelnen Schrittzyklus die Steuerung (31) eine Frühstellungsphase (t11-t12) ermittelt, während welcher eine Ferse mit dem Boden in Kontakt ist und eine Fußzehe nicht mit dem Boden in Kontakt ist, eine Spätstellungsphase (t13-t14) ermittelt, während welcher die Fußzehe mit dem Boden in Kontakt ist und die Ferse mit dem Boden nicht in Kontakt ist, eine Früh-Schwungphase (t14-t15) ermittelt, während welcher ein Bein angehoben ist, und eine Spät-Schwungphase (t15-t16) ermittelt, während welcher das Bein abgesenkt ist;
die Steuerung (31) veranlasst, dass mindestens eine der Elektroden (11, 12, 13) eine elektrische Stimulation während der Frühstellungsphase (t11-t12) ausgibt, oder veranlasst, dass mindestens eine der Elektroden (11, 12, 13) eine elektrische Stimulation während der Frühstellungsphase (t11-t12) nicht ausgibt;
die Steuerung (31) veranlasst, dass mindestens eine der Elektroden (11, 12, 13) eine elektrische Stimulation während der Spätstellungsphase (t13-t14) ausgibt, oder veranlasst, dass mindestens eine der Elektroden (11, 12, 13) eine elektrische Stimulation während der Spätstellungsphase (t13-t14) nicht ausgibt; und
die Steuerung (31) veranlasst, dass mindestens eine der Elektroden (11, 12, 13) eine elektrische Stimulation während der Spät-Schwungphase (t15-t16) ausgibt, oder veranlasst, dass mindestens eine der Elektroden (11, 12, 13) eine elektrische Stimulation während der Spät-Schwungphase (t15-t16) nicht ausgibt.

6. Elektrostimulator (1) nach einem der Ansprüche 1 bis 4, wobei
die Steuerung (31) ausgebildet ist, aus einem Detektor ein Detektiersignal zu empfangen, das sich entsprechend einem Schrittzyklus zyklisch ändert;
die Elektrode eine Elektrode für eine untere Gliedmaße (11-13) ist, die mit der Steuerung (31) verbunden und ausgebildet ist, eine Kniegelenksstimulation unter der Kontrolle der Steuerung (31) auszugeben; und
der Elektrostimulator (1) ferner aufweist
ein computerlesbares Aufzeichnungsmedium, das mit der Steuerung (31) verbunden oder in dieser enthalten ist, wobei das computerlesbare Aufzeichnungsmedium einen computerlesbaren Befehl enthält, der so ausgebildet ist, dass die Steuerung (31):
die Elektrode für die untere Gliedmaße (11-13) veranlasst, eine erste Stimulation des Kniegelenks mit einer ersten Intensität auszugeben, sobald das Detektiersignal eine Mittelstellungsphase (t12-t13) anzeigt; und
die Elektrode für die untere Gliedmaße (11-13) veranlasst, eine zweite Kniegelenksstimulation mit einer zweiten Intensität auszugeben, die niedriger als die erste Intensität ist, oder die Ausgabe einer Kniegelenksstimulation aus der Elektrode für die untere Gliedmaße (11-13) beendet, sobald das Detektiersignal eine Schwungphase (t14-t16) anzeigt.

## Revendications

1. Stimulateur électrique (1) comprenant :
un dispositif de commande (31) qui détermine au moins, dans un cycle de marche unique, une phase à mi-posture (t12-t13), durant laquelle une plante entière de pied se trouve en contact avec le sol, et une phase de basculement (t14-t16), durant laquelle la plante entière de pied ne se trouve pas en contact avec le sol ; et
une électrode (10) qui émet une stimulation électrique au niveau d'un muscle s'étendant à travers une articulation de genou,
**caractérisé en ce que**
le dispositif de commande (31) comprend une période durant la phase à mi-posture (t12-t13) dans laquelle le dispositif de commande (31) a la stimulation électrique émise par l'électrode (10) et une période durant la phase de basculement (t14-t16) dans laquelle le dispositif de commande (31) a l'émission électrique plus faible émise par l'électrode (10) que l'émission de stimulation électrique durant la phase à mi-posture (t12-t13), ou
le dispositif de commande (31) comprend une période durant la phase à mi-posture (t12-t13) dans laquelle le dispositif de commande (31) a la stimulation électrique émise par l'électrode (10) et une période durant la phase de basculement (t14-t16) dans laquelle le dispositif de commande (31) n'a pas la stimulation électrique émise par l'électrode (10).

2. Stimulateur électrique (1) selon la revendication 1,
dans un cycle de marche unique, le dispositif de commande (31) déterminant une phase de posture précoce (t11-t12), durant laquelle un talon se trouve en contact avec le sol et un orteil du pied ne se trouve pas en contact avec le sol, et
le dispositif de commande (31) a la stimulation électrique émise par l'électrode (10) durant la phase de posture précoce (t11-t12).

3. Stimulateur électrique (1) selon la revendication 1 ou 2,
dans un cycle de marche unique, le dispositif de commande (31) détermine une phase de posture tardive (t13-t14), durant laquelle l'orteil du pied se trouve en contact avec le sol et le talon ne se trouve pas en contact avec le sol, et
le dispositif de commande (31) a la stimulation électrique émise par l'électrode (10) durant la phase de posture tardive (t13-t14).

4. Stimulateur électrique (1) selon l'une quelconque des revendications 1 à 3,
dans la phase de basculement (t14-t16), le dispositif de commande (31) détermine une phase de basculement précoce (t14-t15), durant laquelle une jambe est soulevée, et une phase de basculement tardive (t15-t16), durant laquelle la jambe est abaissée, et
le dispositif de commande (31) comprend une période dans laquelle le dispositif de commande (31) a la stimulation électrique émise par l'électrode (10) durant la phase de basculement tardive (t15-t16) et une période dans laquelle le dispositif de commande (31) a la stimulation électrique émise par l'électrode (10) plus faible que l'émission de stimulation électrique durant la phase à mi-posture (t12-t13), ou
le dispositif de commande (31) comprend une période dans laquelle le dispositif de commande (31) a la stimulation électrique émise par l'électrode (10) durant la phase de basculement tardive (t15-t16) et une période dans laquelle le dispositif de commande (31) n'a pas la stimulation électrique émise par l'électrode (10) durant la phase de basculement précoce (t14-t15).

5. Stimulateur électrique (1) selon l'une quelconque des revendications 1 à 4, comprenant une pluralité d'électrodes (11, 12, 13) et une unité de montage (14), où :
l'unité de montage (14) est un objet monté sur un membre inférieur ;
l'une des électrodes (11, 12, 13) est une électrode de muscle ventral (11) fixée à une partie de l'unité de montage (14) correspondant à un groupe de muscles ventraux de membre inférieur, qui est un muscle s'étendant à travers l'articulation du genou ;
l'une des électrodes (11, 12, 13) est une électrode de muscle dorsal (12) fixée à une partie de l'unité de montage (14) correspondant à un groupe de muscles dorsaux de membre inférieur, qui est un muscle s'étendant à travers l'articulation du genou ;
l'une des électrodes (13) est une électrode de muscle gastrocnémien (13) fixée à une partie de l'unité de montage (14) correspondant à un groupe de muscles gastrocnémiens de membre inférieur, qui est un muscle s'étendant à travers l'articulation du genou ;
dans le cycle de marche unique, le dispositif de commande (31) détermine une phase de posture précoce (t11-t12), durant laquelle un talon se trouve en contact avec le sol et un orteil du pied ne se trouve pas en contact avec le sol, une phase de posture tardive (t13-t14), durant laquelle l'orteil du pied se trouve en contact avec le sol et le talon ne se trouve pas en contact avec le sol, une phase de basculement précoce (t14-t15), durant laquelle une jambe est soulevée, et une phase de basculement tardive (t15-t16), durant laquelle la jambe est abaissée ;
le dispositif de commande (31) a au moins une stimulation électrique émise par les électrodes (11, 12, 13) durant la phase de posture précoce (t11-t12) ou n'a pas au moins une stimulation électrique émise par les électrodes (11, 12, 13) durant la phase de posture précoce (t11-t12) ;
le dispositif de commande (31) a au moins une stimulation électrique émise par les électrodes (11, 12, 13) durant la phase de posture tardive (t13-t14) ou n'a pas au moins une stimulation électrique émise par les électrodes (11, 12, 13) durant la phase de posture tardive (t13-t14) ; et
le dispositif de commande (31) a au moins une stimulation électrique émise par les électrodes (11, 12, 13) durant la phase de basculement tardive (t15-t16) ou n'a pas au moins une stimulation électrique émise par les électrodes (11, 12, 13) durant la phase de basculement tardive (t15-t16).

6. Stimulateur électrique (1) selon l'une quelconque des revendications 1 à 4,
le dispositif de commande (31) étant configuré pour recevoir, depuis un détecteur, un signal de détection qui change de manière cyclique selon un cycle de marche ;
l'électrode est une électrode de membre inférieur (11-13) reliée au dispositif de commande (31) et configurée pour émettre la stimulation d'articulation de genou sous la commande du dispositif de commande (31) ; et
le stimulateur électrique (1) comprend en outre
un milieu d'enregistrement pouvant être lu par ordinateur relié à ou incorporé dans le dispositif de commande (31), le milieu d'enregistrement pouvant être lu par ordinateur contenant une instruction pouvant être lue par ordinateur configurée de sorte que le dispositif de commande (31) :
a l'émission d'électrode de membre inférieur (11-13) d'une première stimulation d'articulation du genou ayant une première intensité chaque fois que le signal de détection indique une phase à mi-posture (t12-t13) ; et
a l'émission d'électrode de membre inférieur (11-13) d'une seconde stimulation d'articulation du genou ayant une seconde intensité, qui est inférieure à la première intensité, ou stoppe l'émission d'une stimulation d'articulation de genou depuis l'électrode de membre inférieur (11-13) chaque fois que le signal de détection indique une phase de basculement (t14-t16).
